# EUROPEAN PATENT APPLICATION

(11) **EP 0 813 872 A1**
(43) Date of publication of application: **29.12.1997**
(21) Application number: 97109897.5
(22) Date of filing: 18.06.1997
(51) Int. Cl.: A61K 31/435

(54) **Berberine derivates for inhibiting hsp27 production**

(30) Priority: 18.06.1996 JP 177535/96
(71) Applicant: Kureha Chemical Industry Co., Ltd., Tokyo 103 (JP)
(72) Inventor: Kiyosuke, Yoichi, Tokyo 169 (JP); Tsuduki, Tomoko, Warabi-shi, Saitama 335 (JP); Shirakami, Toshiharu, Kodaira-shi, Tokyo 187 (JP); Morino, Masayoshi, Tokyo 179 (JP); Yoshikumi, Chikao, Kunitachi-shi, Tokyo 186 (JP)
(74) Representative: Cohausz & Florack

(57) **Abstract**

A pharmaceutical composition comprising a berberine derivative of the formula (I): 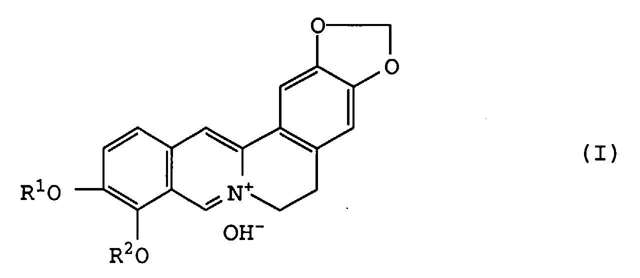 wherein R¹ and R² are independently an alkyl group of 1 to 3 carbon atoms, or a pharmaceutically acceptable salt thereof, as a substance inhibiting production of a protein belonging to an HSP27 family, and a pharmaceutically acceptable carrier, is disclosed. The substance inhibiting production of a protein belonging to an HSP27 family can effectively improve the physiological conditions of a patient suffering from diseases, such as cancer or multiple sclerosis, onset, malignant progression or obstacle to therapy of which is believed to be due in part to the HSP27 family.

## Description

### BACKGROUND OF THE INVENTION

### 1. Field of the Invention

The present invention relates to a pharmaceutical composition containing a berberine derivative or a pharmaceutically acceptable salt thereof as a substance inhibiting the production of a protein belonging to the group of heat shock proteins having a molecular weight of between 16 and 40 kDa (hereinafter referred to as a protein belonging to an HSP27 family).

An agent for inhibiting HSP27 production according to the present invention is able to inhibit the production of the proteins belonging to the HSP27 family in tissues, to thereby effectively improve the physiological condition of patients suffering from diseases such as cancer or multiple sclerosis, the onset, malignant progression or obstacle to therapy of which is believed to be due in part to the HSP27 family, or to effectively treat such diseases.

### 2. Description of the Related Art

Although chemotherapy, surgical therapy, radiotherapy, and immunotherapy recently have made advances, the malignant progression of cancer is still directly or indirectly involved in deaths due to cancer. A successful control of the malignant progression of cancer is one of the most serious problems in cancer treatment. The malignancy of cancer is determined, for example, by a proliferative, invasive or metastatic ability of cancer. Metastasis is a malignant progression phenomenon of cancer, and organs vulnerable to metastasis vary with the kind of primary cancer. The metastasis of cancer is a complicated phenomenon which comprises complex cascades of a proliferation of the primary tumor, a detachment of cancer cells from the primary site, an invasion of the cancer cells into peripheral tissues, a proliferation of the cancer cells therein, tumor angiogenesis, an invasion of cancer cells into adjacent blood vessels, a migration of the cancer cells to distant sites when carried by the bloodstream, an adhesion and implantation of the cells to vascular endothelial cells, an invasion of the cells to the outside of the blood vessel, a commencement of a proliferation at distant sites (metastatic tissues), a new tumor angiogenesis, and a formation of visible metastatic lesions. Generally, cancers include those having a high malignancy and those having a relatively low malignancy. A method of a fundamentally treatment of a highly malignant cancer has not been established, and in most cases, a patient suffering from cancer finally dies.

A hyperthermia treatment of cancer is a method of treating cancer by warming a cancer tissue to thereby selectively kill tumor cells, and has recently attracted attention. The treatment of cancer by hyperthermia has many advantages in that a lethal effect on cells can be obtained at relatively moderate temperature range of 41 to 45 °C, as a biological effect obtained by a moderate heat, and a synergism can be obtained by combining this with radiation or anticancer drugs. The treatment of cancer by hyperthermia is clinically applied in almost all departments of a hospital. One of the problems of the hyperthermia treatment, however, is a thermotolerance transiently induced after a heat treatment. That is, cancer cells become transiently thermotolerant due to the first hyperthermia treatment, and thus the cytocidal effect is lowered in the next hyperthermia treatment. The term thermotolerance means that a cell or tissue becomes transiently resistant to a next heating, by once heating the cell or tissue to a sublethal condition. Taking the thermotolerance into account, the hyperthermia treatment is limited and is carried out only once or twice a week, in almost all medical institutions under the present circumstances.

In the chemotherapy treatment of cancer, there still exist solid tumors, such as lung or colorectal cancers, which give very little response to a chemotherapeutic agent. Further, there is a problem of drug resistance. That is, an anticancer agent finally becomes ineffective on cancers formerly by responsive to a chemotherapeutic agent. Statistics obtained in the U.S.A. in 1988 show that, among the cancers diagnosed for a period of one year, intrinsically resistant cancers that exhibit a resistance to a chemotherapeutic agent from the beginning account for 49 %, whereas acquired resistant cancers that are cured and disappear with initial chemotherapy but recur, account for 47 %. It is apparent therefrom that one of the most important problems in the prevention of this effect of the chemotherapeutic agent on cancer is a resistance to a cytotoxic drug.

Multiple sclerosis (MS) is an inflammatory demyelinating disease in which a specific disorder occurs in the central nervous system white matter, or an autoimmune disease in which an immune system is shown to attack the myelin sheath enveloping nerve fibers in a mechanism of pathogenesis. In many cases, multiple sclerosis repeats an acute exacerbation and remission at an initial phase, but thereafter shows a gradually progressive symptom. Adrenocorticotropic hormone (ACTH) or corticosteroid was used to improve symptoms in the acute stage, or an immunosuppressive agent, such as azathiopurine or cyclophosphamide, was used to prevent a recurrence in the remission or a development of the chronic progressive symptom. Nevertheless, many drugs administered to a patient suffering from multiple sclerosis do not exhibit the expected effect, are non-specific in effect, and exhibit many side effects, and thus are not satisfactory. Therefore, the development of a method for the specific treatment of multiple sclerosis is needed.

Heat shock proteins (HSPs) or stress proteins are a series of proteins expressed in a cell, when the cell is stimulated by a certain stress, such as heat, heavy metals, chemical agents, amino acid analogues, or a low concentration of oxygen. HSPs occur naturally and widely, and are produced in microorganisms, yeasts, plants, insects, and higher animals including humans.

HSPs include many types, and may be generally classified into four families, by molecular weight, i.e., an HSP90 family having an HSP of 90 kDa or 110 kDa; an HSP70 family having an HSP of 70-73 kDa; an HSP60 family having an HSP of 57-68 kDa; and a small HSP family having an HSP of 20 kDa, 25-28 kDa or 47 kDa. In this specification, an HSP having a particular molecular weight is indicated by the term "HSP" followed by a "numerical figure". For example, an HSP having a molecular weight of 27 kDa is indicated by the term "HSP27".

As above, there exist many kinds of HSPs which are different not only in their molecular weights but also in structure, function or properties. Some of these proteins are synthesized constitutively in addition to the response to the stresses, and have been shown to play physiologically essential roles including a folding and unfolding of proteins, assembly of the subunits of proteins, and a membrane transport of proteins, under normal conditions. These functions of the heat shock protein are called a molecular chaperone.

There is a remarkable correlation between the expression of proteins belonging to the HSP27 family and the nodal metastasis, lymphatic/vascular invasion, and the shorter disease-free survival period (J. Natl. Cancer Inst., 83: 170-178, 1991). It has been reported that the expression of the proteins belonging to the HSP27 family seems to be a negative prognostic factor in stomach cancer (Br. J. Surg., 78: 334-336, 1991). Further, it has been reported that the expression levels of the proteins belonging to the HSP27 family in primary cancer cells positively correlate to cancer malignancy, particularly the recurrence rate of cancer (Breast Cancer Res. Treat., 12: 130, 1988; Proc. Am. Assoc. Cancer Res., 30: 252, 1989). Therefore, it is possible to prevent malignant progression of cancer by inhibiting the expression of the proteins belonging to the HSP27 family.

There are reports that the proteins belonging to the HSP27 family are involved in the thermotolerance which is one of the problems of the hyperthermia treatment. Specifically, when a human HSP27 gene was introduced into mouse and Chinese hamster cells and expressed, thermotolerant cells able to survive after a heat shock were induced and increased, depending on the amount of HSP27 (J. Cell. Biol., 109: 7-15, 1989). Further, variants from Chinese hamster cells which constitutively expressed the HSP27 increased the heat-resistance (J. Cell. Physiol., 137: 157, 1988). Furthermore, αB-crystallin is a protein which is induced by a heat shock treatment and has a high homology of an amino acid sequence to the HSP27, and thus, is one of the proteins belonging to the HSP27 family. The cultured glial cells wherein αB-crystallin was expressed in excess gained an increased tolerance to heat stress (J. Cell. Biol., 125: 1385-1393, 1994). This raises the possibility that the thermotolerance can be suppressed and the effect of the hyperthermia treatment on cancer can be enhanced by inhibiting the expression of the proteins belonging to the HSP27 family. It is also reported that there is correlation between the expression of the proteins belonging to the HSP27 family and drug resistance (Breast Cancer Res. Treat., 23: 178, 1992: Cancer Res., 51; 5245-5252, 1991). Therefore, it would be possible to suppress the drug resistance and enhance the effect of the chemotherapy treatment by suppressing the expression of the proteins belonging to the HSP27 family.

It is elucidated that the immunodominant antigen in multiple sclerosis is αB-crystallin (Nature, 375: 739-740, 1995). Further, αB-crystallin is expressed in the nervous tissue of a multiple sclerosis patients more strongly than in the tissue of healthy persons, and thus has an extremely high immunogenicity (Nature, 375: 798-801, 1995). The above facts show that the autoantigen in multiple sclerosis is αB-crystallin, one of the proteins belonging to the HSP27 family, and therefore, the suppression of the expression of αB-crystallin in the myelin sheath brings beneficial results in the essential treatment of multiple sclerosis.

### SUMMARY OF THE INVENTION

The inventors of the present invention engaged in intensive studies into the provision of an agent for inhibiting the production of the proteins belonging to the HSP27 family which can efficiently improve physiological conditions of patients suffering from the diseases, such as cancer or multiple sclerosis, and efficiently treat said diseases.

As a result, the present inventors unexpectedly found that berberine contained in Phellodendri Cortex or Coptidis Rhizoma, a berberine derivative, or a pharmaceutically acceptable salt thereof can specifically inhibit the production of the proteins belonging to the HSP27 family in cells belonging to the tissue exhibiting a pathosis. More particularly, the present inventors found that the production of the proteins belonging to the HSP27 family in a cell can be inhibited by administering the berberine derivative, or the pharmaceutically acceptable salt thereof, whereby diseases, such as cancer or multiple sclerosis, can be treated. The present invention is based on these findings.

The object of the present invention is to provide an agent for inhibiting the production of the protein belonging to the HSP27 family, which agent can efficiently treat the diseases, such as cancer or multiple sclerosis.

Other objects and advantages of the present invention will be apparent from the following description.

The present invention relates to a pharmaceutical composition, particularly an agent for inhibiting production of a heat shock protein having a molecular weight of between 16 and 40 kDa, namely, a protein belonging to an HSP27 family, comprising a berberine derivative of the formula (I): 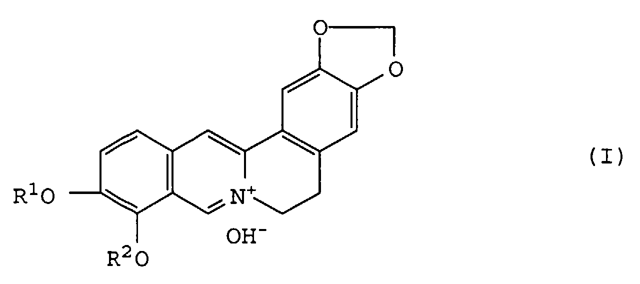 wherein R¹ and R² are independently an alkyl group of 1 to 3 carbon atoms, or a pharmaceutically acceptable salt thereof, as an active ingredient, and a pharmaceutically acceptable carrier or diluent.

Further, the present invention relates to a method for inhibiting production of a protein belonging to an HSP27 family in a mammal, comprising administering to said mammal the berberine derivative of the formula (I) according to claim 1, or a pharmaceutically acceptable salt thereof, in an effective amount for inhibition.

Still further, the present invention relates to a method for treating or preventing cancer or autoimmune disease, comprising administering to a mammal in need thereof the berberine derivative of the formula (I) according to claim 1, or a pharmaceutically acceptable salt thereof, in an effective amount for treatment or prevention.

Still further, the present invention relates to the use of the berberine derivative of the formula (I) or a pharmaceutically acceptable salt thereof, for preparing a pharmaceutical composition, particularly an agent for inhibiting production of the proteins belonging to the HSP27 family.

As shown above, the term "HSP27 family" includes proteins belonging to the group of heat shock proteins having a molecular weight of between 16 and 40 kDa. As examples of the proteins belonging to the HSP27 family, there may be mentioned an HSP27 of mammals, namely a heat shock protein having a molecular weight of 27 kDa, or an HSP28 of mammals, namely a heat shock protein having a molecular weight of 28 kDa; an HSP25 of birds, namely a heat shock protein having a molecular weight of 25 kDa; and an HSP26 of yeast, namely a heat shock protein having a molecular weight of 26 kDa. Note, a molecular weight of a protein generally varies in accordance with the methods of measuring a molecular weight or the experimental conditions, and the proteins, belonging to the HSP27 family include proteins, such as the HSP27 and the HSP28 of mammals. These are indicated by different molecular weights, but it is unclear at present whether they are different proteins having different amino acid sequences or are the same proteins merely having apparently different indications of the molecular weights.

The proteins belonging to the HSP27 family are the principal heat shock proteins in the small HSP family in mammals, and the characteristics thereof are well conserved among species. Nevertheless, the proteins belonging to the HSP27 family include various proteins whose molecular weights are variable (16 kDa to 40 kDa) in different species, unlike other heat shock proteins. Further, αB-crystallin having a high homology in the amino acid sequence with the HSP27 is induced by a heat shock treatment, and is one of the proteins belonging to the HSP27 family.

### DESCRIPTION OF THE PREFERRED EMBODIMENTS

The present invention will be explained in detail hereinafter.

The pharmaceutical composition of the present invention contains the berberine derivative of the formula (I) or the pharmaceutically acceptable salt thereof as an active ingredient, i.e., a substance inhibiting production of the proteins belonging to the HSP27 family. The alkyl group of 1 to 3 carbon atoms includes, for example, methyl, ethyl, n-propyl, or isopropyl group. In the compound of the formula (I), R¹ and R² are independently an alkyl group of 1 to 3 carbon atoms.

Of the berberine derivatives of the formula (I) which may be used as the substance inhibiting production of the proteins belonging to the HSP27 family, according to the present invention, a berberine of the formula (II): 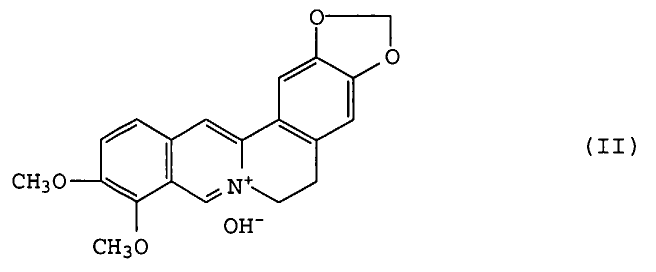 is preferable. The berberine is contained in a crude drug, for example, "Phellodendri Cortex" or "Coptidis Rhizoma".

The pharmaceutically acceptable salts of the berberine derivative which may be used as the substance inhibiting production of the proteins belonging to the HSP27 family according to the present invention include, for example, the pharmaceutically acceptable salts with organic or inorganic acids. As the salts, there may be mentioned, for example, hydrochlorides, sulfates, methanesulfonates, or p-toluenesulfonates, and further, salts with dicarboxylic acids, such as oxalic, malonic, succinic, maleic, or fumaric acid, or monocarboxylic acids, such as acetic, propionic, or butyric acid. The hydrochlorides, such as berberine chloride, i.e., the compound of the formula (III): 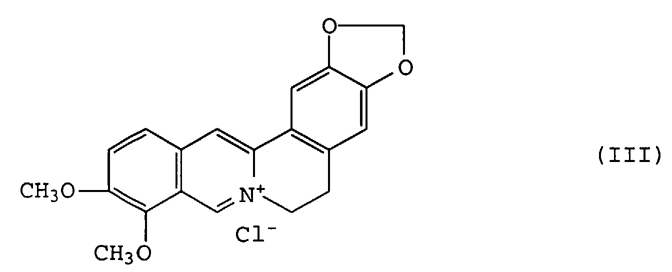 is particularly preferable.

The berberine derivative or the pharmaceutically acceptable salt thereof which may be used as the substance inhibiting production of the proteins belonging to the HSP27 family in the present invention may be prepared by chemical synthesis or extraction of a naturally-occurring material followed by purification of the extract. Alternatively, a commercially available berberine derivative or the pharmaceutically acceptable salt thereof may be used. When the berberine derivative or the pharmaceutically acceptable salt thereof which may be used as the substance inhibiting production of the proteins belonging to the HSP27 family according to the present invention is extracted from a natural material, the whole plant containing the berberine derivatives or the pharmaceutically acceptable salts thereof, or a part of the plant, such as whole grasses, leaf, root, rhizome, stem, root bark or flowers, is extracted without further treatment, or after a simple treatment such as drying, cutting, scalding, heating by steam or pulverizing, to prepare crude drugs. The extraction conditions are not limited as long as they are the conditions generally used for plant extraction. The plant containing berberine is not particularly limited and for example, "Phellodendron amurense Ruprecht" or "Coptis japonica Makino" may be used.

The berberine or the pharmaceutically acceptable salt thereof extracted from a crude drug as the substance inhibiting production of the proteins belonging to the HSP27 family is not particularly limited to, but is preferably extracted from "Phellodendri Cortex" or "Coptidis Rhizoma". "Phellodendri Cortex; Phellodendron Bark" is a bark prepared from Phellodendron amurense Ruprecht or "Rutaceae" by removing periderm. Any parts of the Phellodendri Cortex may be used alone or in a combination thereof. "Coptidis Rhizoma; Coptis Rhizome" is a rhizome prepared from Coptis japonica Makino or "Ranunculaceae" by removing almost all of the root. Any parts of the Coptidis Rhizoma may be used alone or in a combination thereof.

A Phellodendri Cortex or Coptidis Rhizoma extract which may be used as the substance inhibiting production of the proteins belonging to the HSP27 family in the present invention is not limited, as long as it contains the above-mentioned berberine or the pharmaceutically acceptable salt thereof. Thus, a crude Phellodendri Cortex or Coptidis Rhizoma extract may be used as the substance inhibiting production of the proteins belonging to the HSP27 family according to the present invention. Phellodendri Cortex or Coptidis Rhizoma is extracted with, for example, cold water, warm water, or hot water, or an organic solvent, to prepare the Phellodendri Cortex or Coptidis Rhizoma extract which may be used as the substance inhibiting production of the proteins belonging to the HSP27 family according to the present invention. As the organic solvent, there may be mentioned, for example, alcohols of 1 to 6 carbon atoms (such as methanol, ethanol, n-propanol, isopropanol, or butanol), ester (such as methyl acetate, ethyl acetate, propyl acetate, or butyl acetate), ketone (such as acetone, or methyl isobutyl ketone), ether, petroleum ether, n-hexane, cyclohexane, toluene, benzene, organohalogen compound (such as carbon tetrachloride, dichloromethane, or chloroform), pyridine, glycol (such as propylene glycol, or butylene glycol), polyethylene glycol, or acetonitrile. The organic solvents can be used alone or as a mixture of a suitable combination thereof at certain proportions. Further, anhydrous or aqueous organic solvents may be used. Preferably, methanol is used.

As the extraction with water or the organic solvent, an ordinary extraction method for obtaining crude drugs may be used. For example, it is preferable to add 3 to 300 parts by weight of water or organic solvent to 1 part by weight of (dried) Phellodendri Cortex or Coptidis Rhizoma, and then, heat the whole under reflux at a temperature below the boiling point, or to carry out an ultrasonic extraction at an ordinary temperature, or to carry out a cold extraction, with stirring. The extraction procedure may be carried out generally for 5 minutes to 7 days, preferably for 10 minutes to 60 hours. If necessary an auxiliary means, such as stirring, may be used to shorten the extraction time. It is more preferable to defat the crude drugs with, for example, ether or benzene, before the extraction.

The product obtained by extraction with water or the organic solvent may be treated by a suitable process, such as filtration or centrifugation, to remove impurities and obtain a crude extract. The resulting crude extract may be used, without further purification, when the extracted and fractionated berberine derivative or the pharmaceutically acceptable salt thereof obtained from the naturally occurring material is used as the substance inhibiting production of the proteins belonging to the HSP27 family according to the present invention. In addition to the products obtained by an extraction with water or organic solvent, the extract products obtained by treating the above crude extract further with various organic solvents or adsorbents also may be used as the substance inhibiting production of the proteins belonging to the HSP27 family according to the present invention. The Phellodendri Cortex or Coptidis Rhizoma extract which is the crude extract or the various purified extract products may be used in the form of a liquid extract without any treatment other than extraction, a concentrated extract obtained by evaporating the solvents, a pulverized product obtained by evaporation of the solvents and drying, a purified product obtained by crystallization, a viscous product, or a diluted liquid thereof.

The resulting Phellodendri Cortex or Coptidis Rhizoma extracts retain berberine contained in Phellodendri Cortex or Coptidis Rhizoma, or the pharmaceutically acceptable salt thereof, and impurities from the starting Phellodendri Cortex or Coptidis Rhizoma.

It is possible to administer the berberine derivative or the pharmaceutically acceptable salt thereof, or the extract from the plant containing the berberine derivative or the pharmaceutically acceptable salt thereof, such as the extract from the crude drug containing the berberine derivative or the pharmaceutically acceptable salt thereof (particularly, the Phellodendri Cortex or Coptidis Rhizoma extract) according to the present invention, optionally together with preferably a pharmaceutically or veterinarily acceptable ordinary carrier, to an animal, preferably a mammal, particularly humans. The formulation is not limited to but may be, for example, oral medicines, such as powders, fine subtilaes, granules, tablets, capsules, suspensions, emulsions, syrups, extracts or pills, or parenteral medicines, such as injections, liquids for external use, ointments, suppositories, creams for topical application, or eye lotions.

The oral medicines may be prepared by an ordinary method using, for example, fillers, binders, disintegrating agents, surfactants, lubricants, flowability-enhancers, diluting agents, preservatives, coloring agents, perfumes, tasting agents, stabilizers, humectants, antiseptics, antioxidants, or the like, such as gelatin, sodium alginate, starch, corn starch, saccharose, lactose, glucose, mannitol, carboxylmethylcellulose, dextrin, polyvinyl pyrrolidone, crystalline cellulose, soybean lecithin, sucrose, fatty acid esters, talc, magnesium stearate, polyethylene glycol, magnesium silicate, silicic anhydride, or synthetic aluminum silicate. For example, the capsule may be prepared by mixing and filling 1 part by weight of berberine hydrochloride and 99 parts by weight of lactose.

As the parenteral administration, for example, an injection such as a subcutaneous or intravenous injection, or the per rectum administration may be used. This administration by injection is preferable.

When the injections are prepared, for example, water-soluble solvents, such as physiological saline or Ringer's solution, water-insoluble solvents, such as plant oil or fatty acid ester, tonicity agents, such as glucose or sodium chloride, solubilizing agents, stabilizing agents, antiseptics, suspending agents, emulsifying agents or the like may be optionally used, in addition to the berberine derivative or the pharmaceutically acceptable salt thereof, or the extract from the plant containing the berberine derivative or the pharmaceutically acceptable salt thereof, such as the extract from the crude drug containing the berberine derivative or the pharmaceutically acceptable salt thereof (particularly, the Phellodendri Cortex or Coptidis Rhizoma extract), as the substance inhibiting production of the proteins belonging to the HSP27 family according to the present invention.

The pharmaceutical composition may be administered in the form of a sustained release preparation using sustained release polymers. For example, the pharmaceutical composition of the present invention may be incorporated into a pellet made of ethylenevinyl acetate polymers, and the pellet may be implanted in a tissue to be treated.

The amount of the berberine derivative or the pharmaceutically acceptable salt thereof contained in the pharmaceutical composition of the present invention is not limited, in that the pharmaceutical composition may contain 0.01 to 99%, preferably 0.1 to 80% by weight of the berberine derivative or the pharmaceutically acceptable salt thereof.

The pharmaceutical composition of the present invention containing the extract from the plant containing the berberine derivative or the pharmaceutically acceptable salt thereof, such as the extract from the crude drug containing the berberine derivative or the pharmaceutically acceptable salt thereof (particularly, the Phellodendri Cortex or Coptidis Rhizoma extract), as an active ingredient may be prepared by appropriately adjusting the amount of the berberine derivative or the pharmaceutically acceptable salt thereof contained therein to the above range. When the pharmaceutical composition of the present invention containing the above extract is prepared as a pharmaceutical for oral administration, it is preferable to use a pharmaceutically acceptable carrier.

The dose of the pharmaceutical composition of the present invention varies with the kind of disease, the age, sex, body weight, symptoms, method of administration, or the like, but the berberine derivative may be orally or parenterally administered at a dosage of about 1 mg to 10 g a day for an adult, usually divided into one to four dosages.

The composition containing the berberine derivative of the formula (I) or the salt thereof as the substance inhibiting production of the proteins belonging to the HSP27 family may be used not only for the pharmaceutical application but also for various applications, for example, in the form of food such as a functional food or health food.

As explained above, the berberine derivative or the pharmaceutically acceptable salt thereof contained in the present pharmaceutical composition has a function of being able to specifically inhibit the production in cells of the proteins belonging to the HSP27 family. Therefore, when the berberine derivative or the pharmaceutically acceptable salt thereof according to the present invention is administered, the biosynthesis of the proteins belonging to the HSP27 family in cells is specifically decreased. Accordingly, the berberine derivative or the pharmaceutically acceptable salt thereof according to the present invention may be used for treating or preventing cancer, in which the malignant progression thereof involves the proteins belonging to the HSP27 family to enhance the effect of the cancer hyperthermia treatment, which is inhibited by the proteins belonging to the HSP27 family; or for treating or preventing autoimmune diseases, such as multiple sclerosis, an onset in which the proteins belonging to the HSP27 family are involved. Further, it has been reported that there is a correlation between the expression of the proteins belonging to the HSP27 family and drug resistance (Breast Cancer Res. Treat., 23: 178, 1992: Cancer Res., 51; 5245-5252, 1991). Therefore, it would be possible to suppress the drug resistance and enhance the effect of the chemotherapy treatment by suppressing the expression of the proteins belonging to the HSP27 family.

### Examples

The present invention will now be further illustrated by, but is by no means limited to, the following Examples.

### Example 1: Inhibition of HSP production in human cancer cell lines

### (1) Culture of human cancer cell lines

The following various human cancer cell lines were cultured under 5% CO₂, at 37 °C, except for the heat shock treatment period. The lung cancer cell line H69 (ATCC HTB 119), the gastric cancer cell line KATO III (ATCC HTB 103), the colon cancer cell line COLO 205 (ATCC CCL 222), and the prostate cancer cell line DU145 (ATCC HTB 81) were cultured in an RPMI1640 medium containing 10% inactivated fetal bovine serum (hereinafter referred to as FBS).

The uterine cancer cell line HeLa S3 (ATCC CCL 2.2) was cultured in an MEM medium containing 10% inactivated FBS.

The neuroblastoma cell line SK-N-MC (ATCC HTB 10) was cultured in an MEM medium containing non-essential amino acids (L-alanine = 8.9 mg/l, L-asparagine·H₂O = 15.0 mg/l, L-aspartic acid = 13.3 mg/l, L-glutamic acid = 14.7 mg/l, glycine = 7.5 mg/l, L-proline = 11.5 mg/l and L-serine = 10.5 mg/l) and 10% inactivated FBS.

### (2) Treatment with berberine hydrochloride and heat shock treatment

The berberine hydrochloride of the formula (III) (the final concentration = 20 µM: Matsuura Yakugyo) was added to each medium of the above human cancer cell lines 2 days after the inoculation. After the berberine hydrochloride treatment for 24 hours, a heat shock was applied at 45 °C for 15 minutes, and the cells were then cultured at 37 °C overnight. The control tests were carried out by performing the above procedures except that the berberine hydrochloride was not added.

### (3) Determination of amount of HSP produced in human cancer cell lines

The cells treated in the above (2) were homogenized, and the amounts of HSP27 produced were measured by the Western blotting method.

The cells treated in the above (2) were washed with phosphate buffered physiological saline [composition: KC1 = 0.2 g/l, KH₂PO₄ = 0.2 g/l, NaCl = 8 g/1, Na₂HPO₄ (anhydrous) = 1.15 g/l: hereinafter referred to as PBS(-): COSMO BIO CO. Catalogue No. 320-01], and 1 ml of lysis buffer [1.0% NP-40, 0.15 M sodium chloride, 50 mM Tris-HCl (pH 8.0), 5 mM-EDTA, 2 mM-N-ethylmaleimide, 2 mM phenylmethylsulfonyl fluoride, 2 µg/ml leupeptin, and 2 µg/ml pepstatin] was added. The whole was allowed to stand for 20 minutes on ice, and then centrifuged at 4 °C and 12000 rpm for 20 minutes. The resulting supernatant (10 µl) was added to 790 µl of PBS(-), and then 200 µl of a protein assay dye reagent solution (Dye Reagent Concentrate: Bio-Rad Laboratories, Catalogue No. 500-0006) was added. The whole was allowed to stand at room temperature for 5 minutes, and the absorbance at 595 nm was measured to determine the amounts of proteins.

For the SDS-polyacrylamide gel electrophoresis (SDS-PAGE) of the samples, the protein concentration of which were measured, aliquots containing the same amount of protein were loaded onto the gel. The SDS-PAGE was performed according to the Laemmli method (Laemmli, N. K., "Nature", 283: pp.249-256, 1970). After the electrophoresis, the blotting and then blocking were carried out. More particularly, the gel was brought into contact with a 0.45 µm nitrocellulose membrane (Schleicher & Schuell, Catalogue No. 401196) at room temperature and 100 V, using a protein-transferring apparatus (Trans-Blot Electrophoretic Transfer Cell: Bio-Rad), and blotting was carried out for 3 hours. The blotting buffer used was prepared by adding methyl alcohol to Tris-glycine buffer (pH 8.5) consisting of 0.025 M Tris and 0.192 M glycine (Tris Gly Running and Blotting Buffer; Enprotech, Mass., USA; Catalogue No. SA100034), so that the concentration of methyl alcohol became 20%. After blotting, the nitrocellulose membrane was incubated in a 10% skimmed milk-PBS(-) solution at room temperature for 30 minutes, to block non-specific bindings.

After blocking, the first antibody reaction was carried out on the nitrocellulose membrane, using a screener blotter (Sanplatec), and the anti-human-HSP27 mouse monoclonal antibody (StressGen, Victoria, B.C., Canada, Catalogue No. SPA-800). The anti-human-HSP27 mouse monoclonal antibody used was an antibody prepared using, as an immunogen, an HSP24 isolated from human breast cancer cell line MCF7 (ATCC HTB 22) (Cancer Res., 42, 4256-4258, 1982), and reacted specifically with the HSP27 (human HSP27, chimpanzee HSP27, and sheep HSP27) (Cancer Res., 42, 4256-4258, 1982: Cancer Res., 43, 4297-4301, 1983), and specifically with HSP24 and HSP28.

After the first antibody reaction, the nitrocellulose membrane was washed with two fresh PBS(-) solutions for 5 minutes, using a slow rocking shaker, and then with four fresh PBS(-)-0.1% Tween 20 (Bio-Rad, Catalogue No. 170-6531) solutions for 15 minutes, and finally, with two fresh PBS(-) solutions for 5 minutes.

After washing, the second antibody reaction was carried out for 2 hours, using 5 ml of a solution prepared by diluting a peroxidase-labeled goat anti-mouse IgG antibody (CAPPEL, Catalogue No. 55550) 5000-fold with a PBS(-) solution containing 2% skimmed milk. After the reaction, the nitrocellulose membrane was washed with two fresh PBS(-) solutions for 5 minutes, and with five fresh PBS(-)-0.1% Tween 20 solutions for 15 minutes, using a slow rocking shaker, and finally, with two fresh PBS(-) solutions for 5 minutes. After the excess PBS(-) solution was removed, a Western blotting detection reagent (ECL Western blotting detection reagent; Amersham, Catalogue No. RPN2106) was sprinkled over the nitrocellulose membrane, and the membrane was incubated for 1 minute. After the excess detection reagent was removed, the nitrocellulose membrane was enclosed with a wrapping film. The reaction surface was placed in contact with an X-ray film (Kodak X-OMAT, AR; Catalogue No. 165 1454) and exposed. After the film was developed, the presence of HSP27 was detected. The results are shown in Table 1. In Table 1, the symbol "↓" means that the amounts of HSP27 produced were reduced by the berberine hydrochloride-treatment in comparison with the control tests.

**Table 1**

| cancer | cancer cell line | change in amount of HSP27 produced |
|---|---|---|
| uterus | HeLa S3 | ↓ |
| lung | H69 | ↓ |
| stomach | KATO III | ↓ |
| colon | COLO 205 | ↓ |
| prostate | DU145 | ↓ |
| nerve | SK-N-MC | ↓ |

In control tests, i.e., in the case of cell lines which were not treated with the berberine hydrochloride, a single band of a molecular weight of approximately 27 kDa was detected in the uterine cancer cell line HeLa S3, the lung cancer cell line H69, the gastric cancer cell line KATO III, the colon cancer cell line COLO 205, the prostate cancer cell line DU145, and the neuroblastoma cell line SK-N-MC. The molecular weight was determined by molecular weight markers (soybean trypsin inhibitor and bovine carbonic anhydrase) and binding to the anti-human-HSP27 mouse monoclonal antibody. In the case of cell lines treated with the berberine hydrochloride, no band of the molecular weight of approximately 27 kDa was detected in the uterine cancer cell line HeLa S3, the lung cancer cell line H69, the prostate cancer cell line DU145, and the neuroblastoma cell line SK-N-MC. In the gastric cancer cell line KATO III and the colon cancer cell line COLO 205, the detected band of the molecular weight of approximately 27 kDa was faint in comparison with the control tests. Therefore, it can be concluded that the berberine hydrochloride has an activity that will inhibit production of the proteins belonging to the HSP27 family, and is useful as a substance inhibiting production of the proteins belonging to the HSP27 family.

As explained above, the berberine derivative or the pharmaceutically acceptable salt thereof exhibits an activity of inhibiting the production of the proteins belonging to the HSP27 family. Accordingly, by administering the berberine derivative, or the pharmaceutically acceptable salt thereof, the physiological condition of a patient suffering from cancer, in the malignant progression of which the proteins belonging to the HSP27 family are involved, or a suppression of the effect of hyperthermia in which the proteins belonging to the HSP27 family are involved, or the condition of a patient suffering from an autoimmune disease such as multiple sclerosis, an onset in which the proteins belonging to the HSP27 family are involved, may be improved, or said diseases may be effectively treated. Further, it is reported that there is a correlation between the expression of the proteins belonging to the HSP27 family and drug resistance (Breast Cancer Res. Treat., 23: 178, 1992: Cancer Res., 51; 5245-5252, 1991). Therefore, it would be possible to suppress the drug resistance and enhance the effect of the chemotherapy treatment by suppressing the expression of the proteins belonging to the HSP27 family.

Although the present invention has been described with reference to specific embodiments, various changes and modifications obvious to those skilled in the art are deemed to be within the spirit, scope, and concept of the invention.

## Claims

1. A pharmaceutical composition comprising a berberine derivative of the formula (I): 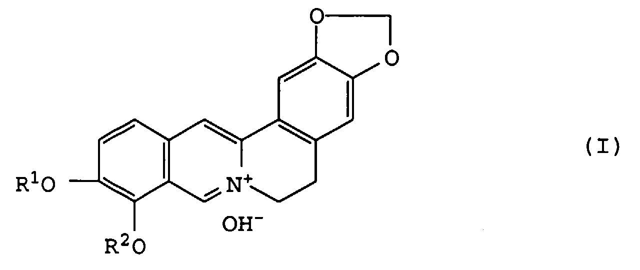 wherein R¹ and R² are independently an alkyl group of 1 to 3 carbon atoms, or a pharmaceutically acceptable salt thereof, as a substance inhibiting production of proteins belonging to an HSP27 family, and a pharmaceutically acceptable carrier.

2. The composition according to claim 1, wherein the berberine derivative is a compound of the formula (II):

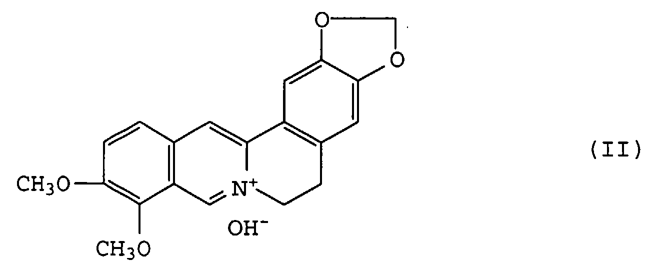

3. The composition according to claim 1, wherein the berberine derivative is a compound of the formula (III):

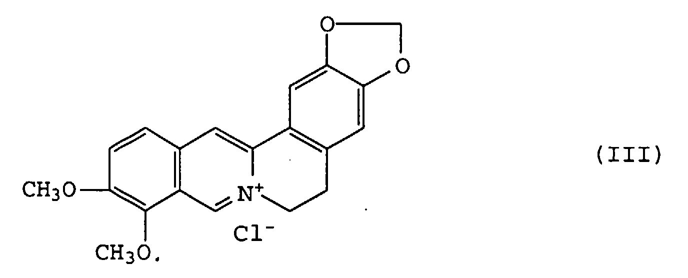

4. A pharmaceutical composition comprising an extract from a plant containing a berberine derivative of the formula (I): 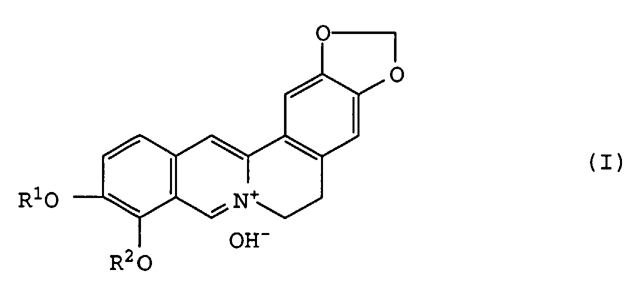 wherein R¹ and R² are independently an alkyl group of 1 to 3 carbon atoms, or a pharmaceutically acceptable salt thereof, as a substance inhibiting production of a protein belonging to an HSP27 family, and a pharmaceutically acceptable carrier.

5. A pharmaceutical composition comprising an extract from the group consisting of a Phellodendri Cortex extract and a Coptidis Rhizoma extract, as the substance inhibiting production of a protein belonging to an HSP27 family, and a pharmaceutically acceptable carrier.

6. Use of the berberine derivative of the formula (I) according to claim 1, or a pharmaceutically acceptable salt thereof, for the preparation of a pharmaceutical composition.

7. A food composition comprising the berberine derivative of the formula (I) according to claim 1.
